# EUROPÄISCHE PATENTSCHRIFT

(11) **EP 2 121 741 B1**
(45) Veröffentlichungstag und Bekanntmachung des Hinweises auf die Patenterteilung: **02.10.2013**
(21) Anmeldenummer: 07856294.9
(22) Anmeldetag: 28.11.2007
(51) Int. Cl.: C07K 14/47, G01N 33/50

(54) **NACHWEIS VON INDIVIDUELLEN T-ZELL-REAKTIONSMUSTERN GEGEN TUMOR-ASSOZIIERTE ANTIGENE (TAA) IN TUMORPATIENTEN ALS BASIS FÜR DIE INDIVIDUELLE THERAPEUTISCHE VAKZINIERUNG VON PATIENTEN**
DETECTION OF INDIVIDUAL T-CELL REACTION PATTERNS AGAINST TUMOR-ASSOCIATED ANTIGENS (TAA) IN TUMOR PATIENTS AS A BASIS FOR THE INDIVIDUAL THERAPEUTIC VACCINATION OF PATIENTS
DÉCÈLEMENT DE MODÈLES INDIVIDUELS DE RÉACTION À CELLULE T VIS-À-VIS D'ANTIGÈNES TUMORAUX (TAA) CHEZ DES PATIENTS ATTEINTS D'UNE TUMEUR, COMME BASE POUR LA VACCINATION INDIVIDUELLE THÉRAPEUTIQUE DE PATIENTS

(30) Priorität: 21.12.2006 DE 102006060824
(43) Veröffentlichungstag der Anmeldung: 25.11.2009
(73) Patentinhaber: Johannes Gutenberg-Universität Mainz, 55122 Mainz (DE)
(72) Erfinder: FATHO, Martina, 55286 Wörrstadt (DE); WESARG, Emmanuelle, 64295 Darmstadt (DE); LENNERZ, Volker, 55270 Ober-Olm (DE); VAN DER BRUGGEN, Pierre, B-1200 Brussels (BE); WÖLFEL, Thomas, 55128 Mainz (DE); DEBO, Serena, 55129 Mainz (DE)
(74) Vertreter: Krauss, Jan
(86) Internationale Anmeldenummer: PCT/EP2007/010329
(87) Internationale Veröffentlichungsnummer: WO 2008/080468

(56) Entgegenhaltungen:
- WO-A-2005/028505
- DE-A1-102005 041 616
- NAIR SMITA K ET AL: "Induction of tumor-specific cytotoxic T lymphocytes in cancer patients by autologous tumor RNA-transfected dendritic cells." ANNALS OF SURGERY APR 2002, Bd. 235, Nr. 4, April 2002 (2002-04), Seiten 540-549, XP002467170 ISSN: 0003-4932 in der Anmeldung erwähnt
- LENNERZ V ET AL: "The response of autologous T cells to a human melanoma is dominated by mutated neoantigens" PROCEEDINGS OF THE NATIONAL ACADEMY OF SCIENCES OF USA, NATIONAL ACADEMY OF SCIENCE, WASHINGTON, DC, US, Bd. 102, Nr. 44, 1. November 2005 (2005-11-01), Seiten 16013-16018, XP002408502 ISSN: 0027-8424
- LIAO XINSHENG ET AL: "Transfection of RNA encoding tumor antigens following maturation of dendritic cells leads to prolonged presentation of antigen and the generation of high-affinity tumor-reactive cytotoxic T lymphocytes" MOLECULAR THERAPY, ACADEMIC PRESS, SAN DIEGO, CA,, US, Bd. 9, Nr. 5, Mai 2004 (2004-05), Seiten 757-764, XP008080544 ISSN: 1525-0016
- JAVOROVIC M ET AL: "RNA transfer by electroporation into mature dendritic cells leading to reactivation of effector-memory cytotoxic T lymphocytes: a quantitative analysis" MOLECULAR THERAPY, ACADEMIC PRESS, SAN DIEGO, CA,, US, Bd. 12, Nr. 4, Oktober 2005 (2005-10), Seiten 734-743, XP002353825 ISSN: 1525-0016
- MIRAN JAVOROVIC: "T-Cell Stimulation by Melanoma RNA-Pulsed Dendritic Cells", DISSERTATION, 15 January 2004 (2004-01-15), pages 1-169, München
- JAVOROVIC M ET AL: "RNA transfer by electroporation into mature dendritic cells leading to reactivation of effector-memory cytotoxic T lymphocytes: A quantitative analysis", MOLECULAR THERAPY 200510 US LNKD- DOI:10.1016/J.YMTHE.2005.03.034, vol. 12, no. 4, October 2005 (2005-10), pages 734-743, ISSN: 1525-0016
- DATABASE BIOSIS [Online] BIOSCIENCES INFORMATION SERVICE, PHILADELPHIA, PA, US Januar 2008 JAVOROVIC MIRAN ET AL: 'Inhibitory effect of RNA pool complexity on stimulatory capacity of RNA-pulsed dendritic cells' Database accession no. PREV200800244446 & JOURNAL OF IMMUNOTHERAPY, vol. 31, no. 1, January 2008 (2008-01), pages 52-62, ISSN: 1524-9557

## Beschreibung

Die vorliegende Erfindung betrifft ein Verfahren zur Identifizierung präferentieller Zielantigene autologer antitumoraler T-Zellantworten bei individuellen Tumorpatienten, umfassend: a) Bereitstellen von isolierten T-Zellen, die nicht durch Kontakt mit einer Tumorzellinie *in vitro* stimuliert sind, aus dem Blut eines Tumorpatienten, b) Bereitstellen von für den Tumorpatienten autologen Dendritischen Zellen (DCs) und/oder B-Lymphocyten (BLCs), wobei die DCs und BLCs vorher mit einer Auswahl von mRNAs transfiziert wurden, die für T-Zell-immunogene TAA kodieren, deren Expression in der Tumorart bekannt ist, und diese exprimieren, c) in Kontakt bringen der T-Zellen mit den DCs und/oder BLCs und d) Identifizieren von denjenigen TAA aus der Auswahl der transfizierten TAA, die in Schritt c) von T-Zellen auf transfizierten DCs und /oder BLCs erkannt werden, mittels eines Stimulations- und Reaktivitätstests. Das Verfahren kann weiterhin die Expansion der T-Zellen, die Antigene der DCs und/oder BLCs erkennen, umfassen. Die vorliegende Erfindung beschreibt weiter ein Verfahren zur Herstellung eines individualisierten Tumorvakzins oder individualisierten Tumortherapeutikums, sowie entsprechende Verfahren zur Behandlung einer Tumorerkrankung unter der Verwendung des individualisierten Tumorvakzins oder individualisierten Tumortherapeutikums.Krebszellen exprimieren eine Vielfalt tumorassoziierter Antigene (TAA). In humanen in vitro-Modellen wurde gezeigt, daß die Erkennung von Oligopeptiden aus TAA durch tumorreaktive cytotoxische T-Lymphozyten (CTL) zur Zerstörung der Tumorzellen führt.CTL erkennen Oligopeptide, die aus cytoplasmatisch abgebauten Proteinen stammen und über Moleküle des Haupt-Histokompatibilitätskomplexes (HLA-Klassen I und II) auf der Zelloberfläche präsentiert werden.

Einen besonderen Ansatz stellt die spezifische, aktive Immuntherapie dar, welche das Immunsystem des Patienten gegen TAA auf den Tumorzellen sensibilisiert und eine Langzeitimmunität erzeugen soll. Im Rahmen der Immuntherapie werden daher Impfstoffe (Vakzine) an den Patienten verabreicht, die entsprechende TAA enthalten. Bisherige Ansätze der Krebsimmuntherapie beschränkten sich auf den Einsatz weniger TAA aus den Kategorien der Differenzierungs- und Cancer/Germline-Antigene (C/G-Antigene), die häufig in Tumorgeweben exprimiert werden und von denen Peptide über die häufigsten in der Bevölkerung vorkommenden HLA-Allele präsentiert werden. Dabei wurde nicht überprüft, ob die Studienpatienten grundsätzlich in der Lage waren, eine Immunantwort gegen die Impfantigene zu entwickeln (Rosenberg et al., Nat. Med. 10: 909, 2004). Von japanischen Arbeitsgruppen wurde versucht, bei Lungenkarzinompatienten und Patienten mit gastrointestinalen Tumoren die Impfung mit Peptiden aus TAA zu individualisieren, indem vorab die peripheren Blutlymphozyten auf Reaktivität gegen Kandidatenpeptide aus TAA getestet wurden. Nur Peptide, gegen die Reaktivität gezeigt werden konnte, wurden für eine Vakzinierung verwendet (Mine et al., Cancer Sci. 94: 548, 2003; Sato et al., Cancer Sci. 94: 802, 2003). Die Auswahl der Kandidatenpeptide aus den TAA beschränkte sich auf HLA-A2- und HLA-A24-bindende Peptide. Zwar sind dies die in asiatischen Bevölkerungsgruppen am häufigsten vorkommenden HLA-Allele, doch ist das Verfahren dadurch nur für HLA-A2- und -A24-positive Patienten zugänglich. T-Zellantworten gegen Peptide aus den gleichen TAA, die über andere HLA-Allele präsentiert werden, können durch die Methode nicht erfaßt werden. Weiterhin beruhte die Methode auf der Stimulation von peripheren T-Zellen der Patienten mit den Kandidatenpeptiden. Inzwischen haben mehrere Untersuchungen gezeigt, daß solche Verfahren zwar zur Expansion peptidspezifischer T-Zellen führen, die T-Zellen aber häufig die Tumorzellen nicht erkennen. Das liegt entweder daran, daß die Tumorzellen das Peptid aus dem TAA nicht prozessieren und präsentieren oder die T-Zellen nur auf die hohen Peptidkonzentrationen, die für die Stimulationen eingesetzt wurden reagieren, nicht aber auf die deutlich geringeren Konzentrationen der natürlich auf den Tumorzellen gezeigten Peptide.

Andere Gruppen verglichen bei Nierenkarzinompatienten nach Nephrektomie das normale Nierengewebe mit dem Tumorgewebe im Hinblick auf Unterschiede in der globalen Expression von Genen und Differenzen in der Präsentation von MHC Klasse I-Peptiden. Die "Gene profiling"-Analysen führten zur Identifizierung von tumorspezifisch exprimierten oder überexprimierten Genen. Die Massenspektrometrie-Untersuchungen der biochemisch aufgereinigten HLA-Peptide isolierten die natürlichen HLA-Liganden der tumorspezifischen oder überexprimierten Proteine (Weinschenk et al., Cancer Res. 62: 5818, 2002). Für das vergleichende "Gene profiling" und die vergleichende Analyse der natürlichen HLA-Peptidliganden von Tumor- und Normalgewebe werden große Mengen beider Gewebe gebraucht. Daher ist dieses Verfahren nur für wenige Tumorarten geeignet und auch nur bei fortgeschrittener Tumorerkrankung anwendbar. Die Autoren der oben zitierten Arbeit schätzen in einem aktuellen Review-Artikel, daß die Sensitivität ihres Verfahrens ihnen erlaubt, von einer Gewebeprobe höchstens 4 % der natürlichen HLA-Liganden zu identifizieren (Rammensee, Immunology and Cell Biology 84: 290,2006). Hinzu kommt, daß nur die wenigsten der auf diesem Weg identifizierten Peptide tatsächlich eine als ausreichend erscheinende Tumorspezifität aufwiesen, und daß nicht klar ist, ob diese Peptide T-Zellantworen auslösen können.Eine weitere Möglichkeit der individualspezifischen Krebsimmuntherapie ist die Impfung der Patienten mit ihren eigenen Tumorzellen oder Produkten der Tumorzellen wie z. B. Hitzeschockprotein/Peptidkomplexen (Srivastava, Curr. Opin Immunol. 18: 201, 2006). Tumorzellen können allein oder gemischt mit dendritischen Zellen injiziert werden (O'Rourke et al., Cancer Immunol. Immunother. 52: 387, 2003). Außerdem besteht die Möglichkeit, die Tumorzellen vor der Applikation durch gentherapeutische Verfahren immunogener zu machen. Die Immunisierung mit Tumorzellen, allein oder gemischt, bzw. fusioniert mit dendritischen Zellen, oder Tumor-Hitzeschockproteinen. an die TAA-Peptide gebunden sind, erfordert größere Mengen Tumormaterials für die Präparation der Vakzine. Dies ist im klinischen Alltag nicht selten ein limitierender Faktor für eine solche Strategie.Für viele Krebsarten wurde gezeigt, dass sie mehrere TAA gleichzeitig exprimieren. Zum Beispiel findet man in 40 % der Brusttumore, 65 % der malignen Melanome und 37-57 % der Lungentumore die simultane Expression mehrerer C/G Antigene (Simpson et al., Nat. Rev. Cancer 5: 615, 2005). Im Fall des Malignen Melanoms exprimieren mehr als 90 % der Tumore zusätzlich Differenzierungsantigene (Boon et al., Ann. Rev. Immunol. 24: 175, 2006).Generell berücksichtigten somit wenige der bisher durchgeführten Impfstudien den individuellen Charakter der Tumor-Wirt-Interaktionen in Krebspatienten.

DE 10 2005 041 616 beschreibt bestimmte Melanom-assoziierte Oligopeptide, die von CD8-positiven cytotoxischen T-Lymphozyten (CTL) als Peptidantigen erkannt werden und die eine CTL-induzierte Lyse und/oder Apoptose von Tumorzellen herbeiführen. Weiterhin betrifft die vorliegende Erfindung die Verwendung dieser Melanom-assoziierten Oligopeptide in der Krebstherapie.

DE 10 2005 013 846 beschreibt eine Strategie für eine Identifizierung und Bereitstellung Tumor-assoziiert exprimierter Antigene und der dafür kodierenden Nukleinsäuren. Diese Strategie beruht auf der Auswertung humaner Protein- und Nukleinsäuredatenbanken im Hinblick auf potenzielle, auf der Zelloberfläche zugängliche, krebsspezifische Antigene. Durch Datamining wird zunächst eine möglichst komplette Liste aller bekannter Gene aufgestellt, die dem Grundprinzip Gen zu mRNA zu Protein folgend auf das Vorhandensein einer oder mehrerer Transmembrandomänen hin untersucht werden. Hieran schließen sich eine Homologiesuche, eine Einteilung der Treffer in gewebsspezifische Gruppen (u.a. Tumorgewebe) und eine Überprüfung der realen Existenz der mRNA an. Schließlich werden die so identifizierten Proteine z.B. durch Expressionsanalysen und proteinchemische Verfahren auf ihre aberrante Aktivierung in Tumoren evaluiert.

Die Kenntnis der präferentiellen Zielantigene des Immunsystems eines Patienten könnte zur Herstellung eines auf den Patienten zugeschnittenen TAA-Impfstoffs führen, der für eine effektive therapeutische Vakzinierung benutzt werden kann. Es ist somit eine Aufgabe der Erfindung, ein Verfahren zur Identifizierung von Tumor-spezifischen T-Zellantworten aus dem peripheren Blut individueller Patienten und ihrer Zielantigene, zur Verfügung zu stellen. Auf Basis dieses Verfahrens sollen auch weitere vorteilhafte Ausführungsformen der Erfindung zur Verfügung gestellt werden.

In einem ersten Aspekt der vorliegenden Erfindung wird diese Aufgabe durch ein Verfahren zur Identifizierung derjenigen TAA eines Tumorpatienten, die T-Zell-immunogen sind, gelöst, wobei das Verfahren a) Bereitstellen von isolierten T-Zellen, die nicht durch Kontakt mit einer Tumorzellinie *in vitro* stimuliert sind, aus dem Blut eines Tumorpatienten, b) Bereitstellen von für den Tumorpatienten autologen Dendritischen Zellen (DCs) und/oder B-Lymphocyten (BLCs), wobei die DCs und BLCs vorher mit einer Auswahl von mRNAs transfiziert wurden, die für T-Zell-immunogene TAA kodieren, deren Expression in der Tumorart bekannt ist, und diese exprimieren, c) in Kontakt bringen der T-Zellen mit den DCs und/oder BLCs und d) Identifizieren von denjenigen TAA aus der Auswahl der transfizierten TAA, die in Schritt c) von T-Zellen auf transfizierten DCs und /oder BLCs erkannt werden, mittels eines Stimulations- und Reaktivitätstests, umfaßt.

Mitchell und Nair (Mitchell und Nair, RNA-transfected dendritic cells in cancer immunotherapy. J Clin Invest. 2000 Nov;106(9):1065-9) und Nair et al. (Induction oftumor-specific cytotoxic T lymphocytes in cancer patients by autologous tumor RNA-transfected dendritic cells. Ann Surg. 2002 Apr;235(4):540-9.) befassen sich mit der Möglichkeit, mit Tumor(-Gesamt)-mRNA ("mRNA encoding the antigenic content of the tumor cell") transfizierte Dendritische Zellen (DC) als Tumorzellsurrogat einzusetzen. Diese Tumorzell-Äquivalente sollen für Vakzinierungen, das Monitoring der entstandenen T-Zellantworten und für die Identifizierung neuer TAA verwendet werden. Außer daß mit den RNA-transfizierten DC die gleichen Antigen-präsentierenden Zellen (APC) benutzt werden, um T-Zellen eines Tumorpatienten *ex vivo* zu stimulieren, hat die vorliegende Erfindung mit der publizierten Methodik nichts gemeinsam. Aus einer Reihe von Patientenmodellen ist bekannt, daß Patienten mit metastasierten Malignomen antitumorale T-Zellantworten im Blut aufweisen. Diese T-Zellen erkennen meistens nur einen sehr kleinen Ausschnitt der vom Tumor exprimierten TAA und im Hinblick auf die eigentlichen Peptidepitope und darauf, über welche HLA-Allele die Peptide erkannt werden, ist die Erkennung hochgradig individualspezifisch.

Viele Methoden des Stand der Technik (auch die in Mitchell und Nair wie oben beschriebene) befassen sich mit der Identifizierung von tumorspezifisch exprimierten Genen, die dann als potentielle *Targets* für die Krebsimmuntherapie dienen sollen. Doch die Expression eines Tumorantigens bedeutet noch nicht, daß es auch immunogen ist. Seine Immunogenität hängt über die Expression hinaus entscheidend davon ab, ob aus dem TAA-Protein Peptide prozessiert und an die individuellen HLA-Moleküle des jeweiligen Patienten gebunden werden können und ob das T-Zellrepertoire des Patienten die HLA-Peptidkomplexe erkennen kann. Daraus resultiert, daß aus mindestens mehreren Dutzend (heute bekannter) potentiell immunogener TAA im individuellen Patienten nur wenige Peptide aus einzelnen TAA tatsächlich eine T-Zellantwort hervorrufen, die aber nur unzureichend vorhersagbar ist. Zur Lösung dieses Problems stellt die vorliegende Erfindung das erfindungsgemäße Stimulations- und Testverfahren zur Verfügung. Es soll diejenigen T-Zellantworten identifizieren, die im Patienten durch Auseinandersetzung mit den Tumorzellen entstanden und aktivierbar sind. Die Reaktivität wird im anschließenden ELISPOT Assay durch die Zytokinfreisetzung bei Antigenkontakt belegt. Nachweisbare T-Zellantworten können dann anschließend gezielt durch eine Vakzinierung verstärkt werden.

Dies ist der wesentliche Unterschied zu allen Vakzinierungsstudien, die bisher durchgeführt wurden. Diese zielten auf die Verstärkung bzw. Generierung von Antworten gegen "Wunschantigene" ab, deren Expression im jeweiligen Tumor zwar gegeben war, aber für die es keine Hinweise darauf gab, ob sie im geimpften Patienten überhaupt immunogen waren. Im Gegensatz zu den Methoden wie oben ist das vorliegende erfindungsgemäße Verfahren tatsächlich unabhängig von der Gewinnung von Tumorzellen aus den Patienten.

Bevorzugt ist ein Verfahren gemäß der vorliegenden Erfindung, das weiterhin die Expansion der T-Zellen, die Antigene der DCs und/oder BLCs erkennen, umfaßt. Weiter bevorzugt ist ein Verfahren gemäß der vorliegenden Erfindung, das weiterhin einen Kontrolltest auf die Reaktivität der T-Zellen gegen das TAA umfaßt. Entsprechende Verfahren sind dem Fachmann bekannt und exemplarisch in den Beispielen unten beschrieben (z.B. IFN-g-ELISPOT-Assay). Ein noch weiter bevorzugtes Verfahren gemäß der vorliegenden Erfindung umfasst weiterhin die Ermittlung der restringierenden HLA Allele, über die erkannte TAA präsentiert werden. Bevorzugte HLA-Allele sind ausgewählt aus Cw16; Cw6; Cw7; Cw2; Cw3; DP4; DR1; DR2; DR3; DR4; DR7; DR11; DR12; DR13; DR15; DR8; DR52; A1; A2; A3; A29; A24; A31; A34; B7; B13; B15; B35; B37; B51; B53; B57; B37; B18; B40; B44; B52; DQ6; DP4; DP1; DP10; und A68. Entsprechende Verfahren sind dem Fachmann bekannt und exemplarisch in den Beispielen unten beschrieben. Das Verfahren der vorliegenden Erfindung weist mehrere Vorteile auf: Im Gegensatz zu früheren ist das Verfahren unabhängig von der Generierung einer stabilen Tumorzelllinie, die zuvor für die Expansion Tumor-spezifischer T-Zellen notwendig war. T-Zellen aus dem peripheren Blut von Tumorpatienten werden statt dessen mit - bevorzugt autologen - dendritischen Zellen (DCs) oder B-Lymphozyten (BLCs) stimuliert, die zuvor mit einer Auswahl ("Panel") von mRNAs transfiziert wurden, die für T-Zell-immunogene Tumor-assoziierte Antigene (TAA) kodieren. Das Panel umfaßt Antigene mehrerer Kategorien, insbesondere Antigene vom Differenzierungs- und vom Cancer/Germline-Typ. Im Rahmen der vorliegenden Erfindung wurden T-Zellen aus dem peripheren Blut von Tumorpatienten in Einzelansätzen mit bis zu 35 immunogenen TAAs in einem Kurzzeitstimulationsprotokoll expandiert. Als Antigen-präsentierende Zellen wurden dendritische Zellen oder B-Zellen des jeweiligen Patienten verwendet, die mit Antigenkodierender mRNA ausgestattet wurden. Die Responder-Lymphozyten dieser Stimulationsansätze wurden auf die Erkennung dieser TAA getestet und es wurden ihre HLA-Restriktionselemente ermittelt.

Der Stimulations- und Reaktivitätstest ermöglicht die Identifizierung eines individuellen Spektrums der Zielantigene antitumoraler T-Zellen unabhängig von der Verfügbarkeit von autologen Tumorzellen. Bisher waren solche Untersuchungen nur in den wenigen Patientenmodellen möglich, in denen eine stabil wachsende Zelllinie aus den jeweiligen Tumoren etabliert werden konnte, die dann für die Stimulation und Expansion Tumor-reaktiver T-Zellen herangezogen wurde. T-Zellen, die entsprechende Antigene aus diesem Panel erkennen, werden bevorzugt expandiert und können z.B. für weitere erfindungsgemäße Verfahren und/oder Pharmazeutika verwendet werden. Das Erkennungsmuster der T-Zellen ist erwartungsgemäß weitgehend spezifisch für den individuellen Patienten; das individuelle Reaktionsmuster wird diktiert durch das Antigen-Expressionsmuster des individuellen Tumors, den individuellen HLA-Phänotyp und der Kapazität des individuellen T-Zellrepertoires, eine antigenspezifische Antwort gegen die jeweiligen HLA-Peptidkomplexe zu generieren. In einem anschließenden Reaktionstest, wie hier beschrieben, kann die Reaktivität der T-Zellen gegen das Stimulationsantigen bestätigt werden, und es können die restringierenden HLA-Allele ermittelt werden, über die erkannte TAA-Peptide präsentiert werden.

In einem weiteren Aspekt des Verfahrens gemäß der Erfindung werden die T-Zellen aus dem peripheren Blut des Tumorpatienten isoliert. Es können jedoch auch andere herkömmliche Quellen an T-Zellen verwendet werden.

Bevorzugt ist ein Verfahren gemäß der vorliegenden Erfindung, wobei die in die DCs oder BLCs transfizierten mRNAs ausgewählt sind aus Antigenen mehrerer Kategorien, nämlich Differenzierungsantigenen, C/G-Antigenen, mutierten Antigenen und überexprimierten Antigenen. Die Erfinder wählten als Antigenformat die Boten-RNA (mRNA), mRNA eignet sich für die transiente Transfektion einer Vielzahl von Zellarten, kodiert die Antigene in voller Länge und schließt daher die Gesamtheit denkbarer Epitope ein. In einigen Vorarbeiten wurde die Eignung von mRNA-transfizierten DCs für den Nachweis von T-Zellantworten überprüft (Britten et al., J Immunol Methods 287:125, 2004; Britten et al., J Immunol Methods 299:165, 2005). Für den geplanten Test werden in vitro transkribierte (IVT) TAA-kodierende mRNAs in mDCs aus peripherem Blut der jeweiligen Patienten transfiziert. RNA-transfizierte DCs werden dann als Stimulatorzellen für die Expansion TAA-reaktiver T-Zellen dienen.

Tumorassoziierte Antigene (TAA) können aufgrund ihres Expressionsmusters in verschiedene Kategorien eingeteilt werden (dazu siehe auch; http://www.cancerimmunity.org/peptidedatabase/Tcellepitopes.htm):
a) Differenzierungsantigene werden nur in Tumoren und Zellen des Gewebetyps, aus dem sie entstehen, exprimiert. Zum Beispiel sind Differenzierungsantigene des Malignen Melanoms (MM) Melanozyten-spezifische Proteine wie Tyrosinase, Tyrosinase-related protein-2 (TRP-2), gp100 und Melan-A/MART-1.
b) "Cancer/Germline"-Antigene (C/G-Antigene, "shared tumor-specific") werden außer in Gameten und Trophoblastzellen in keinem anderen differenziertem Gewebe exprimiert. Epigenetische Veränderungen aufgrund der malignen Transformation führen zur aberranten Expression von C/G-Antigenen in Krebszellen. Meistens exprimieren Tumorzellen mehrere C/G-Antigene gleichzeitig und ihre Expression bleibt im Verlauf der Tumorprogression erhalten. C/G-Antigene werden darüber hinaus in einer Vielzahl von Tumoren verschiedener Histologien exprimiert. c) In der Kategorie mutierte Antigene werden Antigene, die "Missense"-Punktmutationen tragen und Fusionsproteine, die aus tumorspezifischen Translokationen von Gensegmenten entstehen, zusammengefaßt. Bis auf wenige Ausnahmen sind die bisher bekannten punktmutierten Antigene spezifisch für den individuellen Tumor, in dem sie entdeckt wurden. Malignom-spezifische Fusionsproteine wie z.B. BCR/ABL (in Chronisch myelotischen Leukämien) treten dagegen häufig in hämatologischen Krebserkrankungen auf. d) Als vierte Kategorie der TAA treten überexprimierte Antigene in Tumoren auf. Hierzu gehören Proteine, die in Zellen differenzierter Normalgewebe strikt reguliert exprimiert werden, weil viele von ihnen selbst Funktionen wie Wachstum, Zellzyklus, Apoptose und ähnliches regulieren.

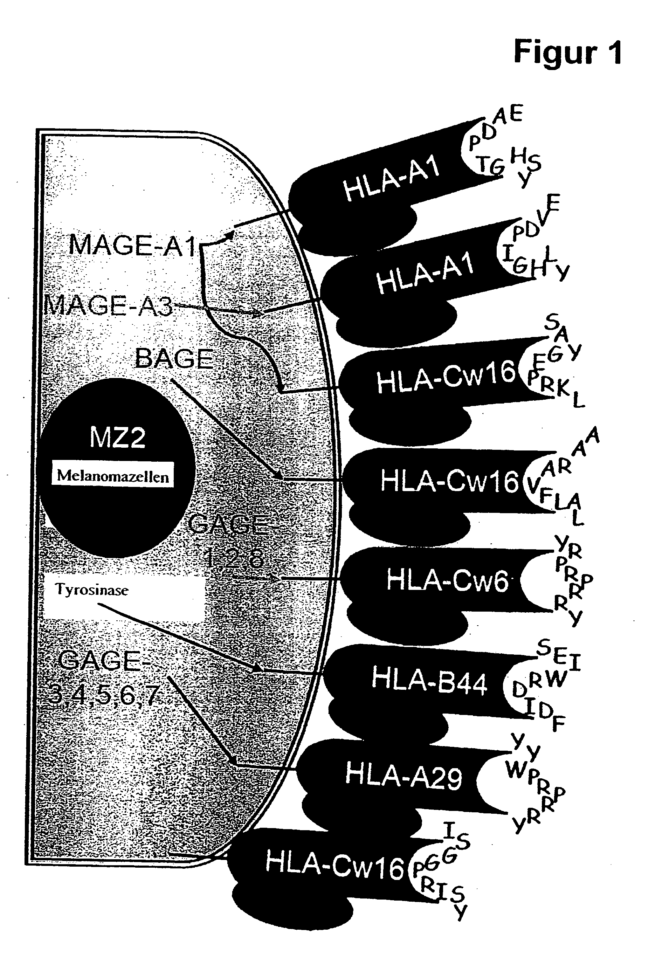

Bevorzugt ist ein erfindungsgemäßes Verfahren, wobei die Differenzierungsantigene aus Melanozyten-spezifischen Proteinen, wie Tyrosinase, Tyrosinase-related protein-2 (TRP-2), gp100 und Melan-A/MART-1 oder C/G-Antigene wie MAGE, GAGE, BAGE ausgewählt sind (vergleiche dazu auch Figur 1). Bevorzugt ist weiter ein erfindungsgemäßes Verfahren, wobei das mutierte Antigen aus einem Fusionsprotein ausgewählt ist, z.B. aus BCR/ABL und anderen bekannten krebsrelevanten Fusionen. In einem weiteren Aspekt des Verfahrens gemäß der Erfindung wird anhand des obigen Verfahrens ein individuelles TAA-Erkennungsmuster durch T-Zellen eines Tumorpatienten identifiziert. Dieses individuelle Antigen-Expressionsmuster dient erfindungsgemäß als wesentliche Basis zur Erzeugung patientenspezifischer ("personalized") Tumorvakzine. Solch ein Vakzin kann auch für ein Antigen-Expressionsmuster hergestellt werden, das erfindungsgemäß aus einer Gruppe an Tumorpatienten identifiziert wird, die an einem bestimmten Tumor erkrankt sind. Damit können bestimmte Tumortypen in einem Patientenkollektiv gezielt behandelt werden. Geeignete Gruppen wären zum Beispiel Tumore bei Nieren-, Brust-, Pankreas-, Magen-, Hoden-, Prostata-, Darm- und/oder Hautkrebs.

In einem weiteren Aspekt der Erfindung ermöglicht der vorgestellte T-Zell-Stimulationstest bei einem breiten Patientenkollektiv die Ermittlung strukturell normaler ("shared") Antigene, die von autologen antitumoralen T-Zellen erkannt werden können. Dabei wird gegen ein umfangreiches Spektrum definierter TAA unter Berücksichtigung der Gesamtheit individueller HLA-Allele getestet. Erst damit wird das volle Potential dieser Antigene ausgeschöpft. Die Kenntnis der präferentiellen Zielantigene des Immunsystems eines Patienten könnte zur Herstellung eines auf den Patienten zugeschnittenen TAA-Impfstoffs führen, der für eine therapeutische Vakzinierung benutzt werden kann. Besonders bevorzugt ist daher ein Verfahren gemäß der Erfindung, wobei das Antigen-Expressionsmuster einer Gruppe an Tumorpatienten identifiziert wird, deren Tumoren strukturell normale ("shared") TAA exprimieren.

Unter dem Begriff "shared antigens" werden solche TAA zusammengefaßt, deren Expression in verschiedenen Tumoren des gleichen Gewebeursprungs (z.B. "Differenzierungsantigene" in Melanomen) oder in Tumoren verschiedener Histologien nachgewiesen wurde. Zu den TAA, die in ganz unterschiedlichen Tumoren exprimiert werden, gehören "überexprimierte Antigene" und so genannte "Cancer/Germline-Antigene" (letztere werden auch als "shared tumor-specific" bezeichnet). Wie gesagt, bezeichnet der Begriff "shared" nur die von verschiedenen Tumoren geteilte Expression der Antigene jedoch nicht ihre Immunogenität. In verschiedenen Melanompatienten wurde nachgewiesen, daß ihre Tumore multiple Antigene aller vier Kategorien (Cancer/Germline-, Differenzierungs-, überexprimierte- und mutierte Antigene) exprimierten. Aber nur ein Teil davon und ganz unterschiedliche TAA waren in den jeweiligen Patienten T-Zell-immunogen. Darüber hinaus war die Spezifität der T-Zellantworten (welches Peptid aus dem TAA wurde über welches HLA-Molekül präsentiert und erkannt) für den jeweiligen Patienten weitgehend spezifisch. Daher sind für die vorgesehenen Untersuchungen alle "shared" Antigene interessant, weil sie potentiell immunogen sind.

Besonders bevorzugte "shared" Antigene im Sinne der vorliegenden Erfindung sind (Positionen in Klammern) BAGE-1; GAGE-1,2,8; GAGE-3,4,5,6,7; GnTV (intron); HERV-K-MEL; KK-LC-1; KM-HN-1; LAGE-1; MAGE-A1; MAGE-A2; MAGE-A3; MAGE-A4; MAGE-A6; MAGE-A9; MAGE-A10; MAGE-A12; MAGE-C2; Mucin; NA-88; NY-ESO-1 / LAGE-2; SAGE; Sp17; SSX-2, SSX-4; und TRP2-INT2 (intron 2).

Beschrieben wird weiterhin ein Verfahren zur Identifizierung eines Tumors in einem Patienten, umfassend zuerst ein Verfahren wie oben genannt und Identifizieren des Tumors basierend auf dem ermittelten Antigen-Expressionsmusters des Tumorpatienten. Es wird weiter ein Verfahren zur Herstellung eines individualisierten Tumorvakzins beschrieben, umfassend ein Verfahren, wie oben genannt, und Formulieren des/der identifizierten TAA/TAAs in Form eines Tumorvakzins. Beschrieben wird weiterhin ein Verfahren zur Herstellung eines individualisierten Tumortherapeutikums, umfassend ein Verfahren wie oben genannt und Formulieren der expandierten identifizierten autologen DCs und/oder BLCs in Form eines Tumortherapeutikums. Aus einer Auswahl prinzipiell immunogener Tumorantigene werden gezielt die herausgefiltert, die im gegebenen Patienten immunogen sind. Diese Antigene können dann bevorzugt in einem Multiepitop-Vakzin für die therapeutische Immunisierung des jeweiligen Patienten eingesetzt werden. Eine therapeutische Vakzinierung mit diesen Antigenen verspricht deutlich bessere klinische Ergebnisse als die Impfung mit Antigenen, von denen nicht klar ist, ob im individuellen Fall eine Immunantwort überhaupt möglich ist. Da der Test ohne Tumorzellen auskommt, ist er generell für all die Patienten einsetzbar, deren Tumoren die Panelantigene oder einen Teil von ihnen exprimieren. Die Unabhängigkeit von Tumorzellen ermöglicht es, den Test auch bei Patienten einzusetzen, die klinisch tumorfrei sind, bei denen aber ein hohes Risiko für eine erneute Progression der Tumorerkrankung besteht. Gerade solche Patienten werden als ideale Kandidaten für die therapeutische Impfung betrachtet.

Die zu behandelnden Tumorerkrankungen umfassen dabei z. B. Nieren-, Brust-, Pankreas-, Magen-, Hoden- und/oder Hautkrebs. Die Aufzählung der Tumorerkrankungen ist dabei lediglich beispielhaft und soll den Verwendungsbereich nicht eingrenzen. Es ist im Stand der Technik bekannt, daß eigens generierte T-Zellen, die spezifisch für bestimmte Peptide waren, effektiv und selektiv Tumorzellen abtöten konnten. Für einen Einsatz von Tumor-assoziierten Antigenen in einer Tumorvakzine sind grundsätzlich mehrere Applikationsformen möglich. So beschrieben Tighe et al (Gene vaccination: plasmid DNA is more than just a blueprint, Immunol. Today 19(2):89-97, 1998), daß das Antigen entweder als rekombinantes Protein mit geeigneten Adjuvantien bzw. Trägersystemen oder als die für das Antigen kodierende cDNA in Plasmidvektoren verabreicht werden kann. In diesen Fällen muß das Antigen im Körper des Patienten von Antigen-präsentierenden Zellen (APC) verarbeitet und präsentiert werden, damit eine Immunantwort ausgelöst wird. Melief et al. (Peptide-based cancer vaccines, Curr. Opin. Immunol. 8:651-657, 1996) zeigten eine weitere Möglichkeit, nämlich die Verwendung von synthetischen Peptiden als Vakzine. Denkbar ist auch die Verabreichung der von T-Zellen erkannten TAA-RNA(s) als (Multiepitop-)Vakzin. RNA kann direkt oder in Form transfizierter DCs eingesetzt werden.

TAA-RNAs oder -Peptide können dabei in einer bevorzugten Ausführungsform mit Zugabe von Adjuvantien verwendet werden, oder aber auch in Alleinstellung. Als Adjuvans kann bspw. der Granulocyte-macrophage-colony-stimulating-factor (GM-CSF) eingesetzt werden. Weitere Beispiele für solche Adjuvantien sind Aluminiumhydroxid, Emulsionen von Mineralölen, wie bspw. das Freund'sche Adjuvans, Saponine oder Siliciumverbindungen. Die Verwendung mit Adjuvantien bietet den Vorteil, daß die ausgelöste Immunantwort verstärkt werden kann und/oder daß das Vakzin stabilisiert wird.

Beschreiben wird außerdem eine pharmazeutische Zusammensetzung, die eines oder mehrere der ermittelten TAA-RNAs oder -Peptide enthält. Diese Zusammensetzung dient bspw. der parenteralen Verabreichung, bspw. subkutan, intradermal oder intramuskulär, oder der oralen Verabreichung. Dabei sind die RNAs oder Peptide in einem pharmazeutisch annehmbaren, vorzugsweise wäßrigen, Träger gelöst oder suspendiert. Darüber hinaus kann die Zusammensetzung Hilfsstoffe, wie bspw. Puffer, Bindemittel, Verdünnungsmittel, etc. enthalten.

RNAs oder Peptide können auch zusammen mit immunstimulierenden Substanzen, z.B. Zytokinen, verabreicht werden. Eine umfassende Darstellung von Hilfsstoffen, wie sie bei einer derartigen Zusammensetzung verwendet werden können, ist bspw. in A. Kibbe, Handbook of Pharmaceutical Excipients, 3. Aufl., 2000, American Pharmaceutical Association and pharmaceutical press, dargestellt. Das Mittel kann dabei zur Prävention, Prophylaxe und/oder Therapie von Tumorerkrankungen eingesetzt werden.

Die in der pharmazeutischen Zusammensetzung vorliegende Menge des Peptids oder der Peptide bzw. der RNA(s)liegt dabei in einer therapeutisch effektiven Menge vor. Dabei können die in der Zusammensetzung enthaltenen Peptide bzw. die von den RNAs kodierten Peptide auch an mindestens zwei verschiedene HLA-Typen binden.

Das Tumorvakzin oder Tumortherapeutikum kann ein personalisiertes Einzelvakzin oder - therapeutikum ein Multiepitop-Vakzin oder -Therapeutikum sein. Das Tumorvakzin oder Tumortherapeutikum enthält somit speziell auf den Patienten oder das Patientenkollektiv abgestimmte TAAs oder T-Zellen, die in der Lage sind, den Tumor oder die Tumore in dem Patienten effektiv zu bekämpfen. Wie bei jeder Form der Individualisierung onkologischer Therapie werden somit die Chancen auf den Therapieerfolg im Einzelfall erhöht.

Beschrieben wird weiter ein Verfahren zur Behandlung einer Tumorerkrankung, umfassend ein Verfahren wie oben genannt und Behandlung der Tumorerkrankung basierend auf dem Antigen-Expressionsmuster wie identifiziert. Zuletzt betrifft die Beschreibung ein Verfahren zur Behandlung einer Tumorerkrankung, umfassend ein Verfahren wie oben genannt und Behandlung der Tumorerkrankung basierend auf dem hergestellten Tumorvakzin oder Tumortherapeutikum. Die zu behandelnden Tumorerkrankungen umfassen dabei wie oben z.B. Nieren-, Brust-, Pankreas-, Magen-, Hoden- und/oder Hautkrebs. Die zur Behandlung benötigte effektive Menge und die Art der Verabreichung kann durch den behandelnden Arzt leicht auf Basis Patienten-spezifischer Parameter ermittelt werden.

Die Erfindung soll nun im Folgenden weiter durch die beigefügten Beispiele verdeutlicht werden, ohne jedoch auf diese beschränkt zu sein. In den Figuren zeigt:
Figur 1: eine Übersicht über die im Patientenmodell MZ2 identifizierten Tumor-assoziierten Antigene. Quellenangaben: MAGE A-1: Traversari C et al., J Exp Med 1992; 176: 1453-7, van der Bruggen P et al., Eur J Immunol 1994a; 24: 2134-40; MAGE A-3 Gaugler B et al. , J Exp Med 1994; 179: 921-30; BAGE Boel P et al., Immunity 1995; 2: 167-75; GAGE 1,2,8 Van den Eynde B et al., J Exp Med 1995; 182: 689-98; GAGE 3,4,5,6,7 De Backer O et al., Cancer Res 1999; 59: 3157-65; Tyrosinase Brichard VG et al., Eur J Immunol 1996; 26: 224-230 und MAGE A-6 Vantomme V et al., Cancer Immun [serial online] 2003; 3: 17.

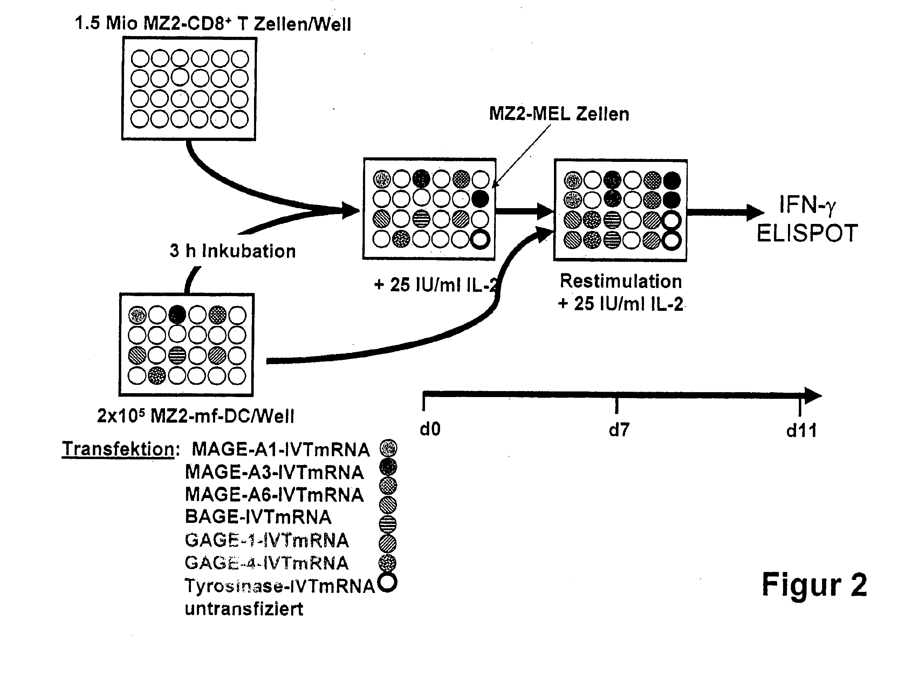

Figur 2: den Stimulationstest in der schematischen Übersicht.

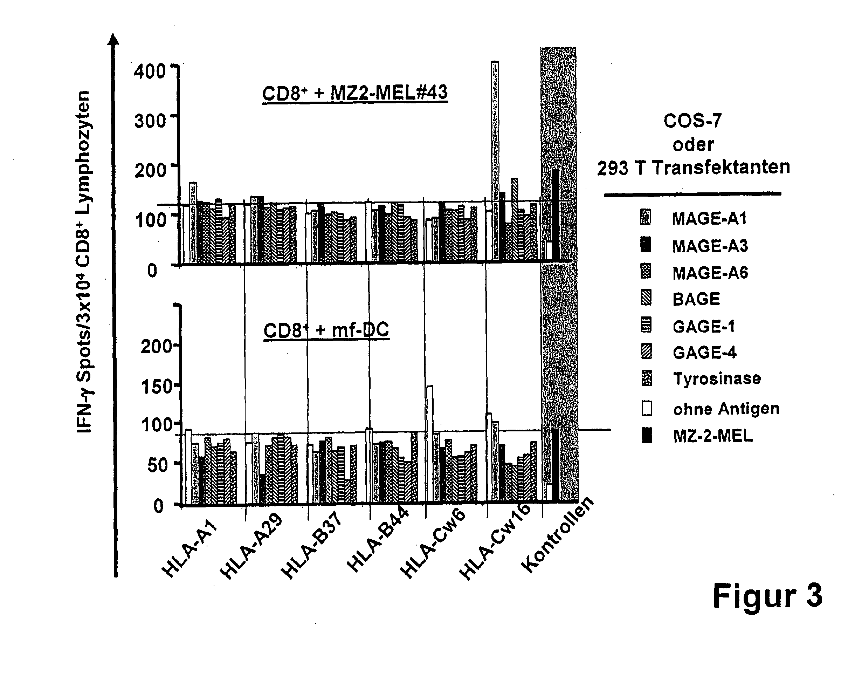

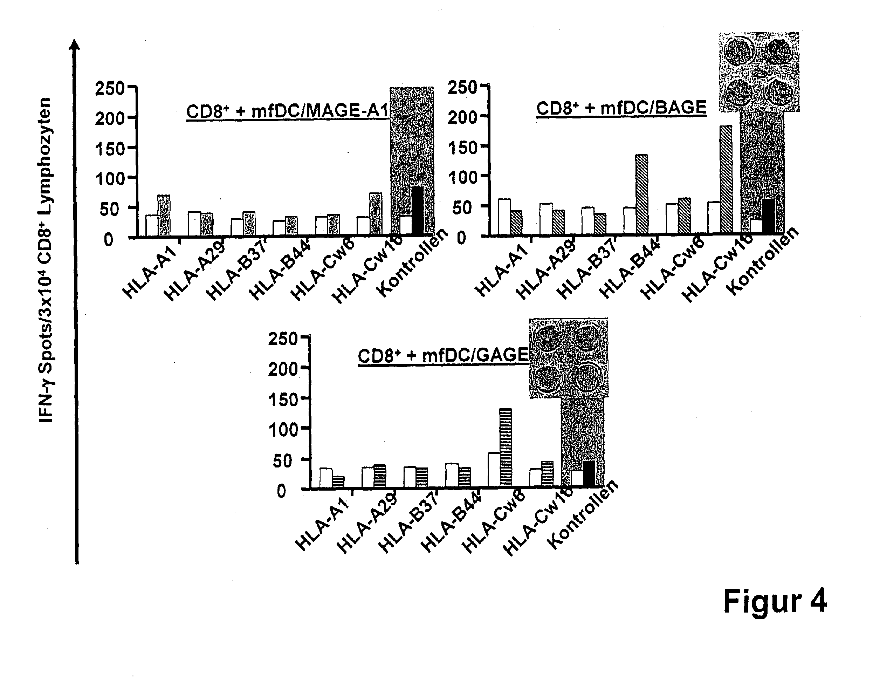

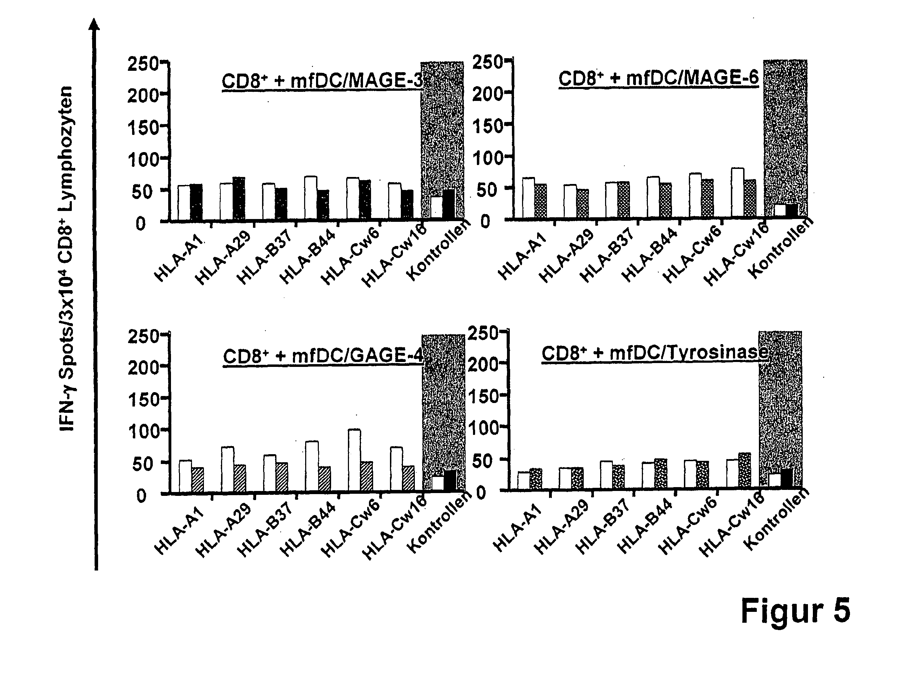

Figuren 3 bis 5: die Reaktionsanalyse der mit Tumorzellen (Figur 3), untransfizierten DC (Figur 3) und mit TAA-RNA transfizierten DC (Figur 4, 5) stimulierten CD8+ T-Zellen von Patient MZ2; mit Tumorzellen stimulierte T-Zellen erkannten nur MAGE-A1/HLA-Cw16-Transfektanten (Figur 3 oben); mit untransfizierten DC stimulierte T-Zellen erkannten keine Transfektanten (Figur 3 unten); T-Zellen, die mit MAGE-A1-transfizierten DC stimuliert wurden, erkannten MAGE-A1/HLA-A1- und MAGE-A1/HLA-Cw16-Transfektanten im Reaktionstest (Figur 4 oben links); T-Zellen, die mit BAGE-transfizierten DC stimuliert wurden, erkannten BAGE/HLA-B44- und BAGE/HLA-Cw16-Transfektanten (Figur 4 oben rechts); T-Zellen, die mit GAGE-1-transfizierten DC stimuliert wurden, erkannten GAGE-1/HLA-Cw6-Transfektanten (Figur 4 unten); von den weiteren stimulierten T-Zellen erkannten keine mehr TAA/HLA-Transfektanten im Reaktionstest (Figur 5).
Figur 6: die Zusammenfassung der Reaktivitäten der durch verschiedene Verfahren generierten T-Zellen gegen alle in diesem Modell gefundenen TAA.

### Beispiele

Im Laufe der letzten Jahre haben die Erfinder in mehreren Tumormodellen von Melanompatienten T-Zell-erkannte TAA entdeckt und charakterisiert. Voraussetzungen dafür waren für jeden Patienten eine stabil wachsende Tumorzelllinie und aus dem peripheren Blut des Patienten isolierte Lymphozyten. Durch Stimulationen der T-Zellen mit der autologen Tumorzelllinie *in vitro* gelang den Erfindern die Generierung von tumorreaktiven T-Zellpopulationen und T-Zellklonen. Diese wurden für die Identifizierung von TAA herangezogen. Dabei zeigte sich, daß von den anttitumoralen T-Zellen jedes der untersuchten Patienten ein individuelles Spektrum von TAA/HLA-Kombinationen erkannt wurde. In jedem Fall wurden neue Peptidepitope von Differenzierungsantigenen, C/G-Antigenen sowie mutierte Antigene erkannt. Nur ausnahmsweise wurden bereits aus der Literatur bekannte Peptid/HLA-Komplexe bestätigt. Offensichtlich führt die Kombination aus tumorspezifischem TAA-Expressionsmuster, dem individuellen HLA-Typ sowie der Fähigkeit des hochvariablen T-Zellrepertoires, auf gegebene Antigen-Kombinationen zu reagieren, zu individualspezifischen Reaktionsmustern. Die Individualität dieser Tumor-Wirt-Interaktion kann derzeit nur in einzelnen Patienten-Modellen analysiert werden, weil es nur von wenigen Patienten gelingt, Tumorzelllinien für die Stimulation der autologen T-Zellen *in vitro* zu generieren. Ohne Stimulation aber lassen sich tumorreaktive T-Zellen aus Patientenblut mit den derzeitigen Methoden nicht nachweisen, weil ihre Frequenzen im peripheren Blut zu niedrig sind. Daher begannen die Erfinder, nach einem Surrogat für Tumorzelllinien für die Stimulation von T-Zellen aus dem peripheren Blut von Tumorpatienten mit TAA zu suchen. Unreife Dendritische Zellen (iDC) können aus peripheren Blut-Monozyten *in vitro* hergestellt werden und zu reifen DC (mDC) differenziert werden, mDC sind professionelle Antigen-präsentierende Zellen, die T-Zellen effektiv stimulieren können. In zwei Melanommodellen, in denen 8 (Modell MZ2) und 13 (D05-GS) T-Zell-erkannte Antigene bekannt waren, transfizierten die Erfinder mDC der Patienten mit den jeweiligen Antigenen und zusätzlichen Kontrollantigenen (D05-GS) und stimulierten T-Zellen aus dem peripheren Blut der Patienten mit den transfizierten DCs. Die Erfinder wählten als Antigenformat die Boten-RNA (mRNA). mRNA eignet sich für die transiente Transfektion einer Vielzahl von Zellarten, kodiert die Antigene in voller Länge und schließt daher die Gesamtheit denkbarer Epitope ein. In einigen Vorarbeiten wurde die Eignung von mRNA-transfizierten DCs für den Nachweis von T-Zellantworten überprüft (Britten et al., J Immunol Methods 287:125, 2004; Britten et al., J Immunol Methods 299:165, 2005). Für den geplanten Test sollten *in vitro* transkribierte (IVT) TAA-kodierende mRNAs in mDCs aus peripherem Blut der jeweiligen Patienten transfiziert werden. RNA-transfizierte DCs sollten als Stimulatorzellen für die Expansion TAA-reaktiver T-Zellen dienen. Letzteres sollte mit IFN-γ-ELISPOT-Assays überprüft werden. Prinzipiell ist es möglich, in jedem Patienten mit einer Tumorart, für die die Expression eines Teils dieser Antigene bekannt ist, mit Hilfe des Stimulationstests T-Zellreaktivitäten gegen TAA zu identifizieren. Erkannte Antigene könnten dann z.B. in Form eines Multiepitop-Vakzins für die therapeutische Immunisierung der Patienten eingesetzt werden.

### Generierung und Transfektion von reifen Dendritischen Zellen von Patienten mit TAA-kodierenden mRNAs

Für die Stimulation der T-Zellen mit RNA-transfizierten DC wurden sowohl "konventionelle" reife DC (mDC für mature DC) als auch so genannte FastDC (mfDC; mature fast DC) verwendet. mDC wurden nach dem Verfahren von Jonuleit et al. (Eur. J. Immunol. 1997; 27 (12): 3135-42) und mfDC nach dem von Dauer et al. (J. Immunol. 2003; 170 (8): 4069-76) aus Monozyten aus den PBMC der Patienten generiert. Für die Transfektion mit TAA-RNA wurden pro Ansatz 2 x 10⁵ DC mit Hilfe des TransMessenger-RNA-Transfektionsreagenz (Qiagen, Hilden) mit 0,8 µg in vitro-transkribierter RNA transfiziert. Danach wurden die Zellen für drei Stunden im Brutschrank inkubiert.

### Stimulation der T-Zellen von Patienten mit den transfizierten DCs

Direkt im Anschluß an die dreistündige Inkubation im Brutschrank wurden die transfizierten DC zur Stimulation der zuvor isolierten CD8⁺ T-Zellen der Patienten eingesetzt. Dabei wurden pro Kultureinheit (KE) einer 24-Loch-Zellkulturplatte 1-1,5 x 10⁶ CD8⁺ T-Zellen mit 1 x 10⁵ transfizierten DC und 2 x 10⁵ CD8-negativen Zellen (so genannten "Feeder-Zellen", bestrahlt mit 100 gray) stimuliert. Als T-Zellwachstumsfaktor wurde humanes rekombinantes Interleukin-2 (IL-2; 25 IU/ml) zugesetzt. Als Kulturmedium wurde AIM-V-Medium (Invitrogen, Karlsruhe), versetzt mit 10% Humanserum (gepooltes Serum gesunder Spender) verwendet. In Kontrollversuchen wurden die T-Zellen mit autologen Melanomzellen (1 x 10⁵) sowie untransfizierten DC stimuliert. An Tag 7 nach Ansetzen des Versuchs wurden die T-Zellen nach dem gleichen Protokoll restimuliert und weitere 4-5 Tage später auf die Erkennung der TAA hin überprüft.

*Nachweis der Reaktivität der T-Zellen gegen die zur Stimulation eingesetzten TAA* gegen die An Tag 11 oder 12 des Versuchs wurden die CD8⁺ T-Zellen auf ihre Reaktivität gegen die Stimulations-TAA und die HLA-Restriktion der Erkennung getestet. Um die unspezifische Reaktivität von z.B. autoreaktiven T-Zellen gegen die DC auszuschließen, wurden für den Reaktionstest 293T-Zellen oder COS-7-Zellen als Antigen-präsentierende Zellen verwendet. Diese wurden mit eukaryontischen Expressionsvektoren transfiziert, die für die TAA kodierende cDNAs enthielten. Jede TAA-cDNA wurde in Einzelansätzen mit der cDNA jedes HLA-Alleles des Patienten ko-transfiziert und die Erkennung der Transfektanten durch die T-Zellen nach 24 Stunden im IFN-γ-ELISPOT-Assay überprüft. Zur Transfektion wurde Lipofectamine 2000 (Invitrogen, Karlsruhe) verwendet. Die Durchführung des Assays erfolgte nach dem in Lennerz et al. (PNAS 2005; 102 (44): 16013-16018) beschriebenen Protokoll. Das vorgestellte Verfahren wurde an zwei gut charakterisierten Patienten-Modellen getestet:

### I: Modell MZ2-MEL

Im Modell MZ2 wurden zuvor acht T-zellerkannte TAA/HLA-Kombinationen identifiziert (Abb. 1): MAGE-A1/HLA-A1 (Traversari C. et al., J. Exp. Med. 1992; 176: 1453-7), MAGE-A3/HLA-A1 (Gaugler B. et al., J. Exp. Med. 1994; 179: 921-30), MAGE-A1/HLA-Cw16 (van der Bruggen P. et al., Eur. J. Immunol. 1994a; 24: 2134-4), BAGE-1/HLA-Cw16 (Boel P. et al., Immunity 1995; 2: 167-75), GAGE-1, 2, 8/HLA-Cw6 (Van den Eynde B. et al., J. Exp. Med. 1995; 182: 689-98), Tyrosinase/HLA-B44 (Brichard V.G. et al., Eur. J. Immunol. 1996; 26: 224-230), GAGE-3, 4, 5, 6, 7/HLA-A29 (De Backer O. et al., Cancer Res. 1999; 59: 3157-65) und MAGE-A6/HLA-Cw16 (Vantomme V. et al., Cancer Immun. [serial online] 2003; 3: 17). CD8⁺ T-Zellen wurden aus PBMC des Patienten isoliert und nach dem oben genannten Verfahren mit DC, die mit jedem der acht TAA transfiziert waren, stimuliert (Abb. 2). Im anschließenden Reaktionstest konnten 4/8 T-Zellspezifitäten nachgewiesen werden (Figuren 4-6). Zusätzlich wurde ein TAA identifiziert, das bisher nicht entdeckt worden war: BAGE-1/HLAB44 (Abb.4, 6). Gleichzeitig wurden die T-Zellen auch mit untransfizierten DC sowie mit den autologen Melanomzellen stimuliert. Während durch die Stimulation mit untransfizierten DC keine TAA-spezifischen T-Zellantworten expandiert wurden führte die Stimulation mit den Melanomzellen zur Erkennung des gleichen TAA-Spektrums wie die Stimulation mit RNA-DC (Tabelle auf Abb. 6).

### II: Modell D05-GS:

In diesem Modell waren zum Zeitpunkt des Stimulationstests 16 T-zellerkannte TAA identifiziert worden. Abweichend vom oben genannten Verfahren wurden in den Versuch anstelle vorisolierter CD8⁺ T-Zellen PBMC eingesetzt. Sonst wurde der Versuch nach dem oben genannten Protokoll durchgeführt: Aliquots der PBMC (1,2 x 10⁶/Ansatz) wurden mit DC, die mit jeder der TAA-RNAs transfiziert waren, sowie mit untransfizierten DC und mit Melanomzellen stimuliert. Nach einer Restimulation an Tag 7 wurden die T-Zellen an Tag 12 in den Reaktionstest eingesetzt. Die mit Melanomzellen stimulierten T-Zellen erkannten 11 der 16 bekannten Antigene (Abb. 7 unten und 10). Mit untransfizierten DC stimulierte T-Zellen erkannten keines der Antigene (Abb. 7 oben), was belegt, daß keine TAA-Reaktivität unspezifisch entsteht. Von den TAA-RNA-stimulierten T-Zellen wurden 4 der bekannten Reaktivitäten wieder erkannt (Figuren 8-10). Zusätzlich wurde eine Spezifität neu entdeckt (MAGE-C2/HLA-A2), die, wie sich in weiteren Untersuchungen herausstellte durch die Stimulation mit dem Tumorzellklon (Klon 6), der für den Melanom-Stimulationsansatz verwendet wurde, nicht zu entdecken war, weil der Tumorklon MAGE-C2 nicht exprimiert. MAGE-C2 wird aber in der Melanomzelllinie, aus der Klon 6 isoliert und mit der der Patient D05-GS über Jahre vakziniert wurde, exprimiert. Dadurch kann die Präsenz der MAGE-C2-reaktiven T-Zellen im peripheren Blut des Patienten erklärt werden. Daß diese T-Zellen durch die Stimulation mit MAGE-C2-RNA transfizierten DC nachgewiesen werden konnten, unterstreicht die Effizienz und Spezifität des Stimulationstests.

## Patentansprüche

1. Verfahren zur Identifizierung der spezifisch erkannten Tumor-assoziierten Antigene (TAA) antitumoraler T-Zellen eines Tumorpatienten, der an einer bestimmten Tumorart erkrankt ist, umfassend:
a) Bereitstellen von isolierten T-Zellen, die nicht durch Kontakt mit einer Tumorzellinie *in vitro* stimuliert sind, aus dem Blut eines Tumorpatienten,
b) Bereitstellen von für den Tumorpatienten autologen Dendritischen Zellen (DCs) und/oder B-Lymphocyten (BLCs), wobei die DCs und BLCs vorher mit einer Auswahl von mRNAs transfiziert wurden, die für T-Zell-immunogene TAA kodieren, deren Expression in der Tumorart bekannt ist, und diese exprimieren,
c) in Kontakt bringen der T-Zellen mit den DCs und/oder BLCs und
d) Identifizieren von denjenigen TAA aus der Auswahl der transfizierten TAA, die in Schritt c) von T-Zellen auf transfizierten DCs und /oder BLCs erkannt werden, mittels eines Stimulations- und Reaktivitätstests.

2. Verfahren nach Anspruch 1, weiterhin umfassend die Expansion der T-Zellen, die Antigene der DCs und/oder BLCs erkennen.

3. Verfahren nach Anspruch 1 oder 2, weiterhin umfassend die Identifizierung der spezifisch erkannten Zielantigene antitumoraler T-Zellen des mindestens einen Tumorpatienten auf Basis der T-Zellen, die Antigene der DCs und/oder BLCs erkennen.

4. Verfahren nach einem der Ansprüche 1 bis 3, weiterhin umfassend einen Kontrolltest auf die Reaktivität der T-Zellen gegen das TAA.

5. Verfahren nach einem der Ansprüche 1 bis 4, weiterhin umfassend die Ermittlung der restringierenden HLA-Allele, über die erkannte TAA präsentiert werden.

6. Verfahren nach einem der Ansprüche 1 bis 5, wobei die T-Zellen aus dem peripheren Blut des Tumorpatienten isoliert werden.

7. Verfahren nach einem der Ansprüche 1 bis 6, wobei die mRNAs ausgewählt sind aus Antigenen mehrerer Kategorien, ausgewählt aus Differenzierungsantigenen, C/G-Antigenen, mutierten Antigenen und überexprimierten Antigenen.

8. Verfahren nach Anspruch 7, wobei die Antigene aus Melanozyten-spezifischen Proteinen oder C/G-Proteinen, wie Tyrosinase, Tyrosinase-related protein-2 (TRP-2), gp 100, MAGE, GAGE, BAGE und Melan-NMART-I ausgewählt sind.

9. Verfahren nach Anspruch 7, wobei das mutierte Antigen aus einem Fusionsprotein ausgewählt ist.

10. Verfahren nach einem der Ansprüche 1 bis 9, wobei ein individuelles Antigen-Expressionsmuster eines Tumorpatienten identifiziert wird.

11. Verfahren nach einem der Ansprüche 1 bis 9, wobei das Antigen-Expressionsmuster einer Gruppe an Tumorpatienten identifiziert wird, die an einem bestimmten Tumor erkrankt sind.

12. Verfahren nach Anspruch 11, wobei der bestimmte Tumor ein malignes Melanom ist.

13. Verfahren nach einem der Ansprüche 1 bis 9, wobei das Antigen-Expressionsmuster einer Gruppe an Tumorpatienten identifiziert wird, die strukturell normale ("shared") TAA exprimieren.

## Claims

1. Method for identifying the specifically recognized tumor-associated antigens (TAA) of antitumoural T-cells of a tumour patient, who is suffering from a certain tumor type comprising:
a) providing isolated T-cells, that are not stimulated by a contact with a tumour cell line *in vitro,* from the blood of a tumour patient,
b) providing dendritic cell (DCs) and/or B-lymphocytes (BLCs) that are autologous for said tumour patient, wherein said DCs and BLCs were transfected beforehand with a selection of mRNAs encoding for T-cell-immunogenic TAA, for which their expression is known in the tumor type, and express these,
c) contacting the T-cells with the DCs and/or BLCs, and
d) identifying such TAA of the selection of transfected TAA that are recognized by T-cells on transfected DCs and /or BLCs in step c), by means of a stimulation- and reactivity test.

2. The method according to claim 1, further comprising the expansion of the T-cells that recognize antigens of the DCs and/or BLCs.

3. The method according to claim 1 or 2, further comprising identifying the specifically recognized target antigens of antitumoural T-cells of the at least one tumour patient on the basis of the T-cells that recognize antigens of the DCs and/or BLCs.

4. The method according to any of claims 1 to 3, further comprising a control assay for the reactivity of the T-cells against the TAA.

5. The method according to any of claims 1 to 4, further comprising determining of the restringing HLA-alleles by which the recognized TAAs are presented.

6. The method according to any of claims 1 to 5, wherein the T-cells are isolated from the peripheral blood of said tumour patient.

7. The method according to any of claims 1 to 6, wherein the mRNAs are selected from antigens of several categories, selected from differentiation antigens, C/G-antigens, mutated antigens, and over-expressed antigens.

8. The method according to claim 7, wherein the antigens are selected from melanocyte-specific proteins or C/G-proteins, such as tyrosinase, tyrosinase-related protein-2 (TRP-2), gp 100, MAGE, GAGE, BAGE, and melan-NMART-I.

9. The method according to claim 7, wherein the mutated antigen is selected from a fusion protein.

10. The method according to any of claims 1 to 9, wherein an individual antigen-expression pattern of a tumour patient is identified.

11. The method according to any of claims 1 to 9, wherein the antigen-expression pattern of a group of tumour patients is identified that are suffering from a specific tumour.

12. The method according to claim 11, wherein the specific tumour is a malign melanoma.

13. The method according to any of claims 1 to 9, wherein the antigen-expression pattern of a group of tumour patients is identified that express structurally normal ("shared") TAAs.

## Revendications

1. Procédé pour l'identification des antigènes associés aux tumeurs (TAA) spécifiquement reconnus par les cellules T anti tumorales d'un patient ayant une tumeur, qui est atteint d'une maladie due à un type de tumeur déterminé, comprenant :
a) la préparation de cellules T isolées, qui ne sont pas stimulées *in vitro* par contact avec une ligne cellulaire tumorale, à partir du sang d'un patient ayant une tumeur,
b) la préparation de cellules dendritiques autologues (DCs) pour le patient ayant une tumeur et/ou de lymphocytes B (BLCs), les DCs et les BLCs ayant été transfectées avec un échantillonnage d'ARNms, qui codent pour les immunogènes TAA de cellules T dont l'expression dans le type de tumeur est connue, et exprimant ceux-ci,
c) la mise en contact des cellules T avec les DCs et/ou les BLCs et
d) l'identification des TAA à partir de l'échantillonnage des TAA transfectés, qui sont reconnus dans l'étape c) sur des DCs transfectées et/ou BLCs, au moyen de tests de stimulation et de réactivité.

2. Procédé selon la revendication 1 comprenant en outre l'expansion des cellules T qui reconnaissent les antigènes des DCs et/ou des BLCs.

3. Procédé selon les revendications 1 ou 2 comprenant en outre l'identification des cellules T anti tumorales des antigènes cibles spécifiquement reconnus d'au moins un patient ayant une tumeur sur la base des cellules T, qui reconnaissent les antigènes des DCs et/ou des BLCs.

4. Procédé selon l'une des revendications de 1 à 3 comprenant en outre un test de contrôle de la réactivité des cellules T vis-à-vis de TAA.

5. Procédé selon l'une des revendications de 1 à 4 comprenant en outre la désignation des allèles HLA récessifs par lesquels les TAA reconnus sont présentés.

6. Procédé selon l'une des revendications de 1 à 5, les cellules T étant isolées à partir du sang périphérique du patient atteint d'une tumeur.

7. Procédé selon l'une des revendications de 1 à 6, les ARNms étant choisis parmi des antigènes de plusieurs catégories, choisis parmi des antigènes de différentiation, des antigènes C/G, des antigènes mutés et des antigènes surexprimés.

8. Procédé selon la revendication 7, les antigènes étant choisis dans le groupe des protéines spécifiques aux mélanocytes ou des protéines C/G, telles que la tyrosinase, la protéine 2 (TRP-2) apparentée à la tyrosinase, la gp 100, la MAGE, la GAGE, la BAGE et la mélan-NMART-I.

9. Procédé selon la revendication 7 l'antigène muté étant choisi à partir d'une protéine de fusion.

10. Procédé selon l'une des revendications de 1 à 9 où le modèle de l'expression de l'antigène individuel d'un patient ayant une tumeur est identifié.

11. Procédé selon l'une des revendications de 1 à 9 où le modèle d'expression de l'antigène d'un groupe de patients ayant une tumeur, qui sont malades d'une tumeur déterminée, est identifié.

12. Procédé selon la revendication 11 où la tumeur déterminée est un mélanome malin.

13. Procédé selon l'une des revendications de 1 à 9 où le modèle d'expression d'un antigène d'un groupe de patients, ayant une tumeur, qui expriment les TAA structurellement normaux (« shared »), est identifié.
